# EUROPÄISCHE PATENTSCHRIFT

(11) **EP 0 887 347 B1**
(45) Veröffentlichungstag und Bekanntmachung des Hinweises auf die Patenterteilung: **30.01.2002**
(21) Anmeldenummer: 98110600.8
(22) Anmeldetag: 10.06.1998
(51) Int. Cl.: C07D 339/04

(54) **Verfahren zur Herstellung trockener Thioctsäure**
Process for the preparation of dry thioctic acid
Procédé pour la préparation d'acide thioctique sec

(30) Priorität: 23.06.1997 DE 19726519
(43) Veröffentlichungstag der Anmeldung: 30.12.1998
(73) Patentinhaber: ASTA Medica Aktiengesellschaft, 01277 Dresden (DE)
(72) Erfinder: Tanner, Herbert, Dr., 63457 Hanau (DE); Drauz, Karlheinz, Prof., 63579 Freigericht-Somborn (DE); Sator, Gerhard, Dr., 64807 Dieburg (DE); Bethge, Horst, 63517 Rodenbach (DE); Möller, Roland, 63546 Hammersbach (DE); Hübner, Frank, Dr., 64372 Ober-Ramstadt (DE); Tacke, Thomas, Dr., 61381 Friedrichsdorf (DE); Rehren, Claus, Dr., 63739 Aschaffenburg (DE)
(74) Vertreter: Wibbelmann, Jobst, Dr., Dipl.-Chem.

(56) Entgegenhaltungen:
- EP-A- 0 487 986
- EP-A- 0 593 896
- EP-A- 0 733 363
- DE-A- 4 229 914

## Beschreibung

Die vorliegende Erfindung betrifft ein Verfahren zur Herstellung trockener Thioctsäure mit der folgenden Formel:

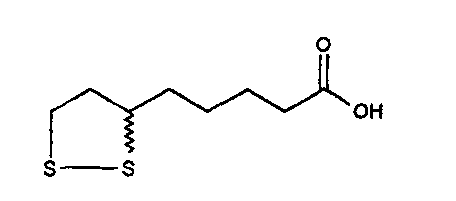

Wenn in der vorliegenden Anmeldung von Thioctsäure die Rede ist, sind immer sowohl die Enantiomeren (R- und S-Form) als auch das racemische Gemisch als auch Mischungen mit beliebigem Verhältnis der Enantiomeren zu verstehen. Die genaue chemische Bezeichnung der Thioctsäure ist 1,2-Dithiolan-3-pentansäure. Das (R)-Enantiomer der Thioctsäure ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z.B. Brenztraubensäure) ist Thioctsäure von essentieller Bedeutung. Thioctsäure ist pharmakologisch wirksam und weist antiphlogistische und antinociceptive (analgetische) sowie zytoprotektive Eigenschaften auf. Eine wichtige medizinische Indikation ist die Behandlung der diabetischen Polyneuropathie.

Thioctsäure wird nach einer Reihe von Verfahren hergestellt, die z.B. in J.S. Yadav et al., J. Sci. Ind. Res. 1990, 49, 400, A.G. Tolstikov et al., Bioorg. Khim. 1990, 16, 1670, L. Dasaradhi et al., J. Chem. Soc., Chem. Commun. 1990, 729, A.S. Gopalan et al., J. Chem. Perkin Trans. 1 1990, 1897, A.S. Gopalan et al., Tetrahedron Lett. 1989, 5705, EP 0487986 A2, EP 0 733 363 A1, E. Walton et al. J. Am. Chem. Soc. 1955, 77, D.S. Acker und W.J. Wayne, J. Am. Chem. Soc. 1957, 79, 6483, L.G. Chebotareva und A.M. Yurkevich, Khim.-Farm. Zh. 1980, 14, 92, beschrieben sind. Das erhaltene Rohprodukt wird dann nach üblichen Verfahren aufgearbeitet und schließlich zum fertigen Arzneimittel verarbeitet. Bei der Zulassung des Arzneimittels werden besonders hohe Anforderungen an die Reinheit des Wirkstoffs und der Zusätze gestellt, u.a. darf der Restgehalt an Lösemittel einen bestimmten Wert nicht überschreiten. In letzter Zeit gibt es Bestrebungen, diesen Grenzwert bei der Thioctsäure noch weiter herabzusetzen.

Der Erfindung liegt somit die Aufgabe zugrunde, ein einfaches technisches Verfahren zu schaffen, mit dessen Hilfe eine Thioctsäure erhalten werden kann, die gegenüber dem Stand der Technik wesentlich verminderte Restgehalte an Lösemittel enthält.

Die genannte Aufgabe wird erfindungsgemäß durch ein Verfahren gelöst, bei dem man ein Rohmaterial, in dem Thioctsäure angereichert vorhanden ist, mit flüssigem oder überkritischem CO₂ behandelt. Das kann batchweise oder kontinuierlich geschehen, wobei das Rohmaterial je nach Fahrweise der Aufarbeitung von dem CO₂ entweder durchströmt oder umspült wird. Die eingesetzte Thioctsäure kann aus einem beliebigen Verfahren zur Herstellung derselben, an das sich gegebenenfalls eine übliche Kristallisation und /oder Extraktion anschließt, stammen. Üblicherweise wird Thioctsäure aus einem Gemisch von Cyclohexan und Essigester kristallisiert. Die auskristallisierte Thioctsäure wird dann abgesaugt und oder zentrifugiert. Das erhaltene Produkt kann dann als Rohmaterial für das erfindungsgemäße Verfahren dienen. Alternativ kann aber auch das teilweise oder überwiegend von Lösemitteln und Verunreinigungen befreite, aber nicht kristallisierte oder extrahierte Produkt des Herstellungsverfahrens verwendet werden.

Auf diese Weise kann man eine Thioctsäure erhalten, die weniger als 1100 ppm, vorzugsweise weniger als 700 ppm, Cyclohexan und weniger als 250 ppm, vorzugsweise weniger als 130 ppm, Essigester enthält, wobei die Nachweisgrenze für Cyclohexan bei 8 ppm und für Essigester bei 3 ppm liegt. Die angeführten Lösemittelgehalte werden am ehesten mit lösungsmittelfeuchtem, nicht thermisch vorgetrocknetem Rohmaterial erhalten. Durch Auswahl geeigneter Bedingungen können auch Verunreinigungen aus dem Herstellungsverfahren in dem CO₂ gelöst und dadurch eine Abreicherung von Nebenprodukten erreicht werden.

Die Verwendung von CO₂ hat den Vorteil, daß dessen kritischer Punkt von 31,1°C und 73,8 bar in einem technisch leicht zugänglichen Temperatur- und Druckbereich liegt. Zusätzlich weist CO₂ die Vorteile auf, daß es unbrennbar ist und nicht die Umwelt gefährdet. Weiterhin kann es leicht von dem Lösemittel getrennt und wiederverwendet werden, indem man das bei der Behandlung anfallende Gemisch von CO₂, Lösemittel und gegebenenfalls Verunreinigungen entspannt, wobei das Lösemittel kondensiert und die Verunreinigungen sich in fester Form abscheiden, sowie anschließend das CO₂ abläßt oder nach Kompression dem Verfahren wieder zuführt. Bei letzterem ist es möglich, mit sehr wenig, in einem Kreisprozeß zurückgeführtem CO₂ zu arbeiten.

Eine bevorzugte Ausführungsform der Erfindung besteht demnach in einem Verfahren, bei dem festes Rohmaterial mit flüssigem oder überkritischem CO₂ durchströmt und/oder umspült wird. Auf diese Weise findet eine Art Fest-Flüssig-Extraktion statt, bei der das Lösemittel aus dem Rohmaterial extrahiert und in dem flüssigem oder überkritischen CO₂ gelöst wird.

Alternativ wird das Rohmaterial in einem Lösemittel gelöst einem Behälter zugeführt und gleichzeitig im Gegenstrom flüssiges oder überkritisches CO₂ durch den Behälter geleitet. Dabei bildet sich ein Konzentrationsgradient des CO₂ in dem Gemisch Thioctsäure/Lösemittel/CO₂ aus, wobei mit zunehmendem CO₂-Gehalt die Löslichkeit der Thioctsäure abnimmt, bis diese auskristallisiert und sich an der Behälterwand niederschlägt. Hierdurch kann der Gehalt an Cyclohexan auf besonders geringe Werte unterhalb 20 ppm veringert werden. Besonders wirtschaftlich kann diese Variante so gestaltet werden, daß man aus dem Herstellungsverfahren erhaltenes Produkt ohne weitere Aufarbeitung in einem Lösemittel oder einem Lösemittelgemisch löst und die erhaltene Lösung als Rohmaterial einsetzt, so daß die bisher übliche, aufwendige Kristallisation mit anschließendem Absaugen und Zentrifugieren entfällt.

Die feste, gereinigte Thioctsäure wird bei beiden Varianten, Extraktion und Kristallisation, durch eine geeignete Vorrichtung zurückgehalten, so daß sie nicht mit dem CO₂-Strom mitgerissen wird.

Das Verfahren wird erfindungsgemäß bei einer Temperatur im Bereich von 20 bis 50°C durchgeführt. Unterhalb 20°C dauert die Trocknung zu lange, so daß die Witschaftlichkeit des Verfahrens beeinträchtigt wird, während bei einer Temperatur oberhalb 50°C die Neigung der Thioctsäure zur Polymerisation zu hoch wird.

Gemäß einer weiteren bevorzugten Ausführungsform der Erfindung wird die Behandlung mit flüssigem oder überkritischem CO₂ bei einem Druck im Bereich von 50 bis 1000 bar durchgeführt. Die Obergrenze dieses Bereichs regibt sich daraus, daß die Löslichkeit der Thioctsäure in CO₂ mit zunehmendem Druck steigt. In den meisten Fällen, insbesondere bei Temperaturen von 35 bis 45°C reicht ein Druck im Bereich von 50 bis 150 bar aus, um die gewünschten Lösemittelgehalte zu erreichen.

Die Gasrate, mit der das eingesetzte Rohmaterial von flüssigem oder überkritischem CO₂ behandelt wird, liegt zwischen 2 und 40 kg CO₂ pro kg Produkt pro Stunde, besonders bevorzugt sind 5 bis 20 kg CO₂ pro kg Produkt pro Stunde. Die Gesamtmenge an CO₂, mit der das Rohmaterial behandelt wird, ist dabei abhängig von der Art und Menge der Verunreinigungen, die entfernt werden sollen. Sie liegt üblicherweise bei 3 bis 30 kg CO₂ pro kg eingesetztem Rohmaterial.

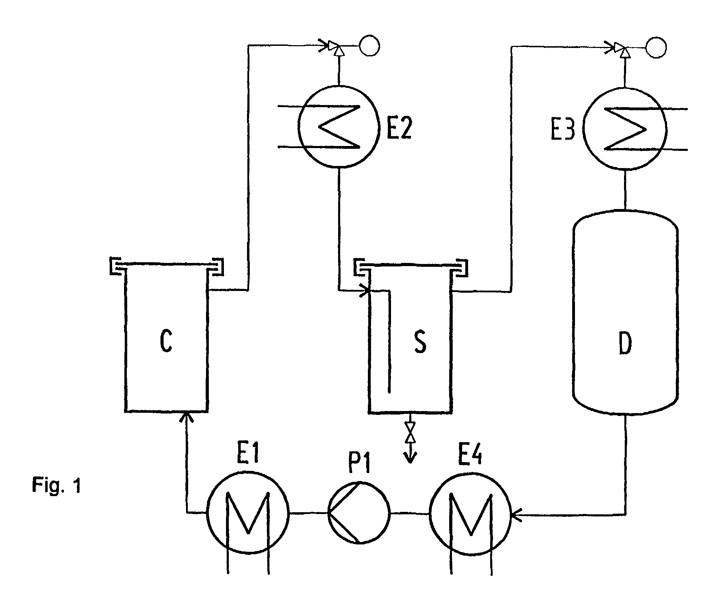

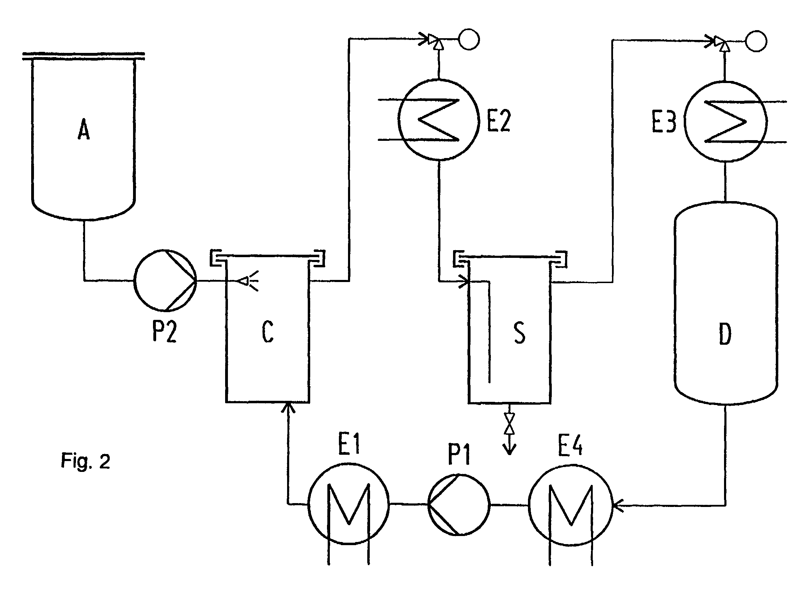

Beispielhafte Vorrichtungen zur Durchführung des oben beschriebenen Verfahrens sind in Fig. 1 und 2 dargestellt.

Die Fig. 1 zeigt eine Anlage zur Extraktion. In dem Gefäß C wird das Rohmaterial vorgelegt. Flüssiges CO₂ aus dem Sammelbehälter D strömt zur Pumpe P 1, die das CO₂ auf Extraktionsdruck komprimiert und es durch den Wärmeaustauscher E 1, in dem es auf Extraktionstemperatur erwärmt wird, in den Extraktor bzw. in die Extraktionskolonne fördert Auf dem Weg durch das Gefäß C lösen sich die extrahierbaren Stoffe im CO₂. Das mit den gelösten Stoffen beladene CO₂ wird zum Separator S geleitet, der vorzugsweise mit einem hier nicht gezeigten Extraktabscheider versehen ist. Durch Änderung von Druck und/oder Temperatur wird die Lösungsfähigkeit des CO₂ im Separator verringert, so daß die Extrakte dort abgeschieden werden. Die Abscheidung kann in mehreren Stufen erfolgen, so daß man Extraktfraktionen unterschiedlicher Qualitäten erhält. Das gasförmige CO₂ aus dem Separator S wird in einem gekühlten Kondensator verflüssigt und im Sammelbehälter D aufgefangen.

### Fig. 2

Die Fig. 2 gibt eine Anlage zur Kristallisation wieder. Die Bauteile C, D, E 1 bis 4, P und S entsprechen denen der Fig. 1. Zusätzlich sind noch ein Vorratsbehälter A und eine Pumpe P 2 vorgesehen. In dem Vorratsbehälter A befindet sich eine Lösung des Rohmaterials, die im Unterschied zu der Anlage von Fig. 1 kontinuierlich über eine Pumpe P 2 dem Gefäß C zugeführt wird. Die Führung des CO₂ erfolgt in gleicher Weise wie in Fig.1, so daß sich ein Gegenstrom zwischen der Lösung des Rohmaterials und dem CO₂ ausbildet.

Für die Messung der Gehalte an Restlösemittel wird die erhaltene Thioctsäure in Methanol gelöst und direkt in einen Gaschromatographen eingespritzt. Der Nachweis erfolgt durch Flammen-lonisations-Detektion (FID) gegen einen internen Standard. Die Nachweisgrenzen betragen im einzelnen:
- Cyclohexan: 8 ppm
- Essigester: 3 ppm

Die nachfolgenden Beispiele erläutern die Erfindung, ohne sie zu beschränken.

### Beispiele 1 bis 7

Es wurden zwei Chargen aus der Thioctsäureproduktion als feuchter Feststoff eingesetzt, die aus einem Gemisch von Cyclohexan und Essigester kristallisiert waren und einen Restgehalt von 15 % Lösemittel aufwiesen. Von diesen Chargen wurden jeweils 3 kg in das Extraktionsgefäß eingebracht. Anschließend wurde die Thioctsäure von unten nach oben mit flüssigem oder überkritischem CO₂ durchströmt, wobei das CO₂ kontinuierlich in den Separator abgelassen wird. Nach Beendigung des Extraktionsvorgangs wurde das trockene Produkt entnommen. Die Versuchsbedingungen sind in der folgenden Tabelle 1 gezeigt, die Ergebnisse einer Analyse der erhaltenen Produkte in Tabelle 2.

**Tabelle 1:**

| Versuchsbedingungen | | | | | |
|---|---|---|---|---|---|
| Beispiel | Charge | T (°C) | Gasrate (kg/h) | Druck (bar) | CO₂-Menge (kg) |
| 1 | 1 | 25 | 20 | 90 | 30 |
| 2 | 1 | 30 | 20 | 90 | 30 |
| 3 | 1 | 40 | 20 | 90 | 30 |
| 4 | 1 | 40 | 20 (30)* | 350 (90)* | 30 |
| 5 | 1 | 40 | 20 | 90 | 30 |
| 6 | 2 | 40 | 20 | 90 | 30 |
| 7 | 2 | 40 | 20 | 90 | 30 |

| | | | | | |
|---|---|---|---|---|---|
| * Die Gasrate betrug anfänglich 20 kg/h und der Druck 350 bar. Nach einem Durchsatz von 10 kg CO₂ wurde 30 Minuten ohne CO₂-Zufuhr stehen gelassen sowie anschließend die Gasrate auf 30 kg/h erhöht und der Druck auf 90 bar vermindert. | | | | | |

**Tabelle 2:**

| Analysenergebnisse | | | | | |
|---|---|---|---|---|---|
| Beispiel | Polymere* (%) | Freie SH-Gruppen (%) | Restlösemittel (ppm) | | Schüttdichte (kg/l) |
| 1 | n.n. | 0,07 | CH: 1010 | EE: 190 | 0,474 |
| 2 | n.n. | 0,08 | CH: 1030 | EE: 197 | 0,486 |
| 3 | n.n. | 0,08 | CH: 491 | EE: 83 | 0,598 |
| 4 | n.n. | 0,08 | CH: 630 | EE: 118 | 0,600 |
| 5 | n.n. | 0,08 | CH: 487 | EE: 92 | 0,555 |
| 6 | 0,24 | 0,09 | CH: 463 | EE: 105 | 0,594 |
| 7 | 0,20 | 0,09 | CH: 522 | EE: 123 | 0,571 |

| | | | | | |
|---|---|---|---|---|---|
| * Bestimmt durch HPLC n.n. = nicht nachweisbar CH = Cyclohexan EE = Essigester | | | | | |

Vergleichsweise können mit einer herkömmlichen Trocknung im Kegelschnecken-Mischtrockner oder Doppelkonus-Mischtrockner nur Cyclohexangehalte von 1500 bis 3000 ppm und Essigestergehalte von 500 bis 1500 ppm erreicht werden. Man erkennt, daß die Extraktion zu einer wesentlichen Verbesserung führt.

### Beispiel 8

Es wurde eine Lösung von 500 g Thioctsäure der Charge 2 in 834 g Essigester hergestellt und im Sammelbehälter vorgelegt. Die Lösung wurde dann in zwei gleich großen Portionen nacheinander unter CO₂-Gegenstrom in das Trocknungsgefäß eingedüst. Hierbei herrschten in dem Gefäß folgende Bedingungen:

| | | |
|---|---|---|
| 1. Portion | Temperatur | 32 bis 36 °C |
| | Druck | 74 bis 90 bar |
| | Gasrate | 5 kg/h |
| | Menge CO₂ | 3 kg |
| | Dauer | 23 min |
| 2. Portion | Temperatur | 34 °C |
| | Druck | 69 bis 78 bar |
| | Gasrate | 10 kg/h |
| | Menge CO₂ | 6 kg |
| | Dauer | 21 min |

Anschließend wurde die Gasrate auf 20 kg/h und die Temperatur auf 40 °C erhöht und bei einem Druck von 90 bar unter diesen Bedingungen eine Stunde nachextrahiert. Im Gefäß schlug sich kristalline Thioctsäure nieder, die einen sehr niedrigen Gehalt an Restlösemittel aufwies, wie in Tabelle 3 gezeigt ist. Mit der Kristallisation läßt sich demnach eine annähernd cyclohexanfreie Thioctsäure in hoher Reinheit gewinnen.

**Tabelle 3**

| Analysenergebnisse | | | | |
|---|---|---|---|---|
| Beispiel | Polymere∗ (%) | Freie SH-Gruppen (%) | Restlösemittel (ppm) | Schüttdichte (kg/l) |
| 8 | 0,34 | 0,08 | CH: 8 | 0,474 |
| | | | EE: 225 | |

Die erhaltene Thioctsäure kann problemlos zu fertigen Arzneimittelformulierungen verarbeitet werden.

## Patentansprüche

1. Verfahren zur Herstellung trockener Thioctsäure, bei dem ein Rohmaterial, in dem Thioctsäure angereichert vorhanden ist, mit flüssigem oder überkritischem CO₂ behandelt wird.

2. Verfahren nach Anspruch 1, bei dem festes Rohmaterial mit flüssigem oder überkritischem CO₂ durchströmt und/oder umspült wird.

3. Verfahren nach Anspruch 2, bei dem das Rohmaterial extrahiert wird.

4. Verfahren nach Anspruch 1, bei dem ein in Lösung vorliegendes Rohmaterial im Gegenstrom mit flüssigem oder überkritischem CO₂ behandelt wird.

5. Verfahren nach Anspruch 4, bei dem das Rohmaterial kristallisiert wird.

6. Verfahren nach Anspruch 5, bei dem aus einem Lösemittelgemisch kristallisiert wird, das sich aus Lösemitteln des Herstellungsverfahrens für das Rohmaterial und flüssigem oder überkritischem CO₂ zusammensetzt.

7. Verfahren nach einem der Ansprüche 1 bis 6, bei dem das flüssige oder überkritische CO₂ nach dem Durchströmen undloder Umspülen des Rohmaterials entspannt wird, mitgerissenes Lösemittel und Verunreinigungen des Rohmaterials in einem Auffangbehälter gesammelt werden sowie das entspannte CO₂-Gas anschließend wieder komprimiert und erneut zur Aufarbeitung bereitgestellt wird.

8. Verfahren nach einem der Ansprüche 1 bis 7, bei dem die Behandlung mit flüssigem oder überkritischem CO₂ bei einer Temperatur im Bereich von 20 bis 50°C durchgeführt wird.

9. Verfahren nach einem der Ansprüche 1 bis 8, bei dem die Behandlung mit flüssigem oder überkritischem CO₂ bei einem Druck im Bereich von 50 bis 1000 bar durchgeführt wird.

10. Verfahren nach einem der Ansprüche 1 bis 9, bei dem die Behandlung mit flüssigem oder überkritischem CO₂ bei einer Gasrate im Bereich von 2 bis 40 kg CO₂ pro kg Produkt pro Stunde durchgeführt wird.

11. Verfahren nach einem der Ansprüche 1 bis 10, bei dem 3 bis 30 kg CO₂ pro kg Rohmaterial verwendet wird.

12. Thioctsäure, die einen Cyclohexan-Gehalt von 20 ppm oder weniger aufweist.

## Claims

1. A process for the production of dry thioctic acid, in which a raw material, in which concentrated thioctic acid is present, is treated with liquid or supercritical CO₂.

2. The process according to claim 1, in which liquid or supercritical CO₂ flows through and/or around solid raw material.

3. The process according to claim 2, in which the raw material is extracted.

4. The process according to claim 1, in which a raw material present in solution is treated in a countercurrent with liquid or supercritical CO₂.

5. The process according to claim 4, in which the raw material is crystallized.

6. The process according to claim 5, in which the crystallization is performed from a solvent mixture composed of solvents of the production method for the raw material and of liquid or supercritical CO₂.

7. The process according to one of claims 1 to 6, in which the liquid or supercritical CO₂ is expanded after flowing through and/or around the raw material, entrained solvent and impurities of the raw material are collected in a catch container and the expanded CO₂ gas is then compressed again and made available again for workup.

8. The process according to one of claims 1 to 7, in which the treatment with liquid or supercritical CO₂ is carried out at a temperature in the range of 20 to 50°C.

9. The process according to one of claims 1 to 8, in which the treatment with liquid or supercritical CO₂ is carried out at a pressure in the range of 50 to 1000 bar.

10. The process according to one of claims 1 to 9, in which the treatment with liquid or supercritical CO₂ is carried out at a gas rate in the range of 2 to 40 kg CO₂ per kg product per hour.

11. The process according to one of claims 1 to 10, in which 3 to 30 kg CO₂ per kg raw material are used.

12. Thioctic acid with a cyclohexane content of 20 ppm or less.

## Revendications

1. Procédé de préparation d'acide thioctique sec dans lequel une matière première dans laquelle l'acide thioctique est présent à l'état enrichi est traitée avec CO₂ liquide ou supercritique.

2. Procédé selon la revendication 1 dans lequel la matière première solide est traversée et/ou balayée par CO₂ liquide ou supercritique.

3. Procédé selon la revendication 2 dans lequel la matière première est extraite.

4. Procédé selon la revendication 1 dans lequel une matière première en solution est traitée à contre-courant avec CO₂ liquide ou supercritique.

5. Procédé selon la revendication 4 dans lequel la matière première est cristallisée.

6. Procédé selon la revendication 5 dans lequel on cristallise dans un mélange de solvants qui se compose de solvants du procédé de préparation pour la matière première et de CO₂ liquide ou supercritique.

7. Procédé selon l'une des revendications 1 à 6 dans lequel le CO₂ liquide ou supercritique est détendu après la traversée et/ou le balayage de la matière première, le solvant entraîné et les impuretés de la matière première sont rassemblés dans un récipient récepteur et le gaz CO₂ détendu est ensuite comprimé de nouveau et préparé de nouveau pour le retraitement.

8. Procédé selon l'une des revendications 1 à 7 dans lequel le traitement avec CO₂ liquide ou supercritique est mis en oeuvre à une température dans le domaine de 20 à 50°C.

9. Procédé selon l'une des revendications 1 à 8 dans lequel le traitement avec CO₂ liquide ou supercritique est mis en oeuvre à une pression dans le domaine de 50 à 1000 bar.

10. Procédé selon l'une des revendications 1 à 9 dans lequel le traitement avec CO₂ liquide ou supercritique est mis en oeuvre à un débit de gaz dans le domaine de 2 à 40 kg de CO₂ par kg de produit par heure.

11. Procédé selon l'une des revendications 1 à 10 dans lequel on utilise 3 à 30 kg de CO₂ par kg de matière première.

12. Acide thioctique qui présente une teneur en cyclohexane de 20 ppm ou moins.
